# EUROPEAN PATENT SPECIFICATION

(11) **EP 3 752 260 B1**
(45) Date of publication and mention of the grant of the patent: **16.08.2023**
(21) Application number: 18715801.9
(22) Date of filing: 16.02.2018
(51) Int. Cl.: A61B 5/00, A61B 5/01, A61B 5/024, A61B 5/103, A61B 5/11, A63B 71/12, A63B 71/06, G16H 20/30, A63B 24/00

(54) **DEVICE FOR MONITORING SPORTS PERFORMANCE, IN PARTICULAR SOCCER PERFORMANCE**
VORRICHTUNG ZUR ÜBERWACHUNG DER SPORTLEISTUNG, INSBESONDERE DER FUSSBALLLEISTUNG
DISPOSITIF DE SURVEILLANCE DE PERFORMANCES SPORTIVES, EN PARTICULIER DES PERFORMANCES DE FOOTBALL

(43) Date of publication of application: 23.12.2020
(73) Proprietor: Soccerment S.r.l., 20121 Milan (IT)
(72) Inventor: COMI, Aldo, 20063 Cernusco Sul Naviglio, Milan (IT); ZAGO, Matteo, 20144 Milano (IT)
(74) Representative: Luppi Intellectual Property S.r.l.
(86) International application number: PCT/IB2018/050956
(87) International publication number: WO 2019/158977

(56) References cited:
- EP-A1- 2 654 030
- EP-A2- 2 809 232
- WO-A1-2017/081555
- WO-A1-2017/120063
- WO-A2-2010/065836
- WO-A2-2010/098589

## Description

### BACKGROUND OF THE INVENTION

The present invention relates to a device suitable for monitoring sports performance, and particularly, but not exclusively, for monitoring soccer performance.

### PRIOR ART

Various devices are known that monitor sports performance.

Electronic clocks have existed for some time that are combined or not with chest bands by wireless connection, worn by athletes during the sports performance, which detect the position, horizontal and vertical motions, heart rate, time taken and other parameters that are useful for monitoring the sports performance. In this manner, the athlete can evaluate his sports performance both during and after the performance. These devices can be used in different sports sectors, like running, cycling, swimming, skiing, canoeing, climbing and others.

Bands exist that are provided with different sensors that enable biomedical, static and dynamic parameters to be detected of the athlete who wears them, and which are in wireless communication with remote stations such as computers and the like, to monitor the sports performance. The biomedical parameters can be heart rate, respiratory rate and the temperature of the skin of the athlete. The static parameters can relate to the posture of the athlete and the dynamic parameters can relate to the motion of the athlete.

In the soccer sector, with reference to a document WO2017/095956, a shin guard is provided that includes tactile devices and sensors of various type, which is in wireless connection with an external computer managed by a trainer, such that the trainer can transmit to the player wearing the shin guard tactile signals in function of the parameters detected by the sensors.

Still in the soccer sector, with reference to a document KR20100032273, a shin guard with sensor is provided that transmits biometric data of the soccer player to an external station, and with reference to a document KR101337821, a shin guard with sensor is provided that transmits vital data on the soccer player to an external station and sends the electric stimuli to the soccer player controlled by the external station in function of the vital data received.

WO2010/065836A2 relates to systems and methods for sensing and monitoring various athletic performance metrics, e.g., during the course of a game, a practice, a training session, training drills, and the like.

EP2809232 relates to sensing devices including flexible and stretchable fabric-based pressure sensors, which may be associated with and incorporated in garments intended to be worn against a body surface; systems and methods for storing, communicating, processing analysing and displaying data collected by sensor components for remote monitoring of conditions at body surfaces, or within the body, are also disclosed.

### OBJECT OF THE INVENTION

One object of the present invention is to propose a monitoring device that is able to monitor the athletic gesture of the soccer player, with particular reference to the technical gesture of the soccer player.

### SHORT DESCRIPTION OF THE INVENTION

This object is achieved by a monitoring device according to claim 1.

The preamble of claim 1 refers to WO2010/065836A2.

### SHORT DESCRIPTION OF THE DRAWINGS

In order to better understand the invention, a description is given below of a nonlimiting exemplary embodiment thereof, illustrated in the attached drawings in which:
fig. 1 is a perspective view of a monitoring device according to the invention;
fig.2 is a front view of the monitoring device of fig. 1 combined with an external device;
fig.3 shows in a perspective view the first of two components of the monitoring device of fig. 1;
fig.4 shows in a front view the component of fig. 3;
fig.5 shows, in a front and rear view, a part of the second component of the monitoring device of fig.1;
fig.6 shows, in an exploded perspective view, the aforesaid second component;
fig.7 shows, in a front and rear view, the aforesaid second component assembled.

### DETAILED DESCRIPTION OF THE INVENTION

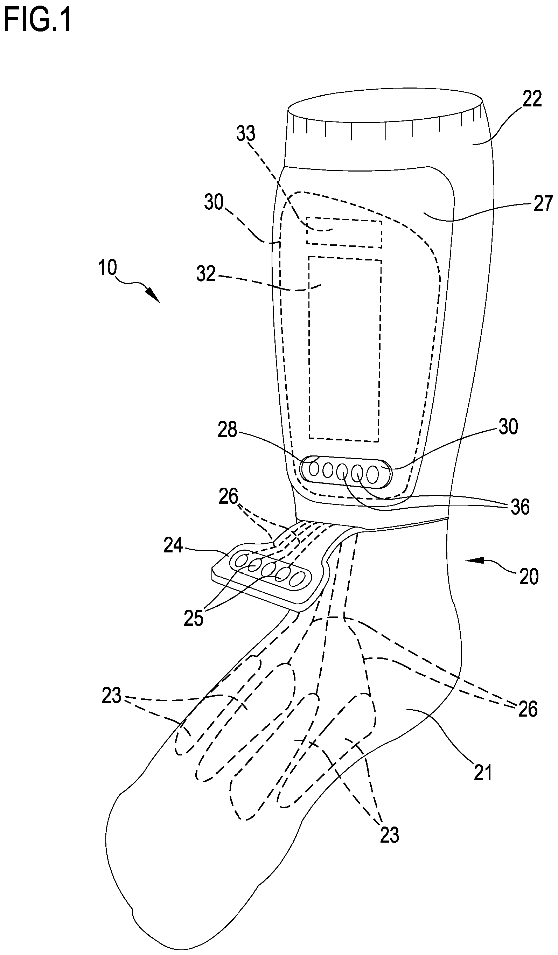

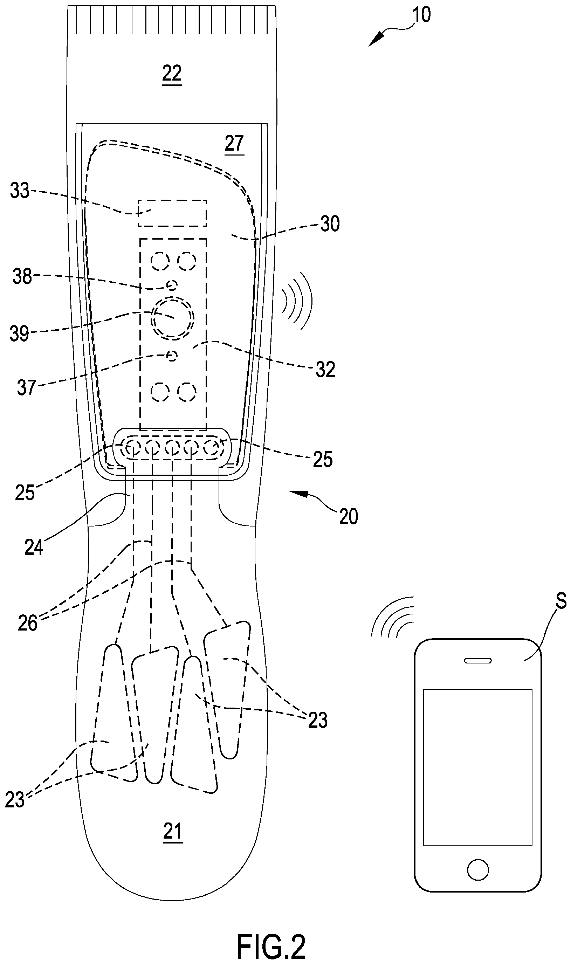

The monitoring device illustrated in figs 1,2 is indicated generally with 10.

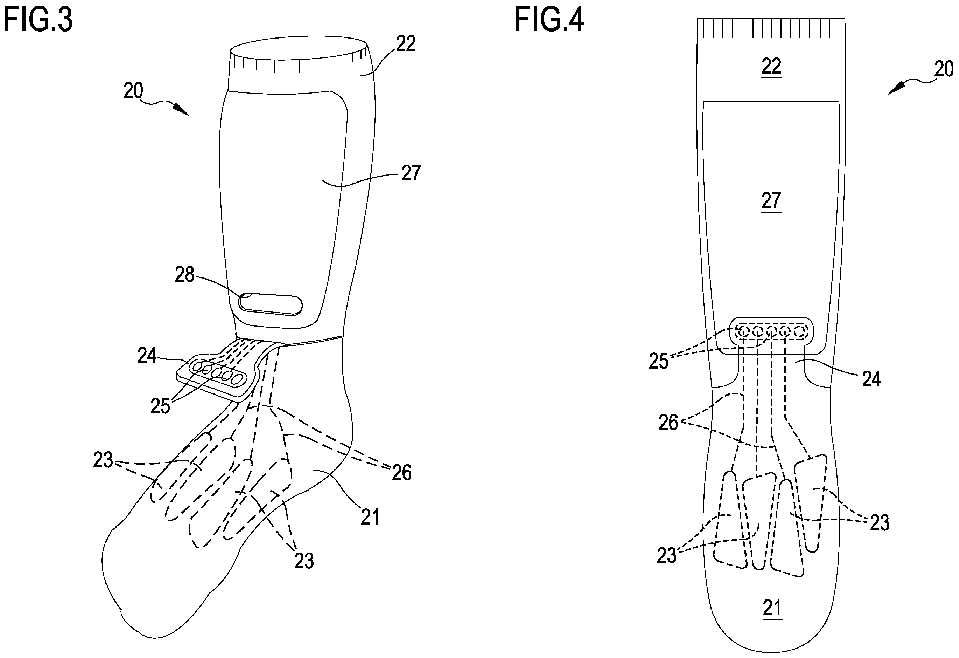

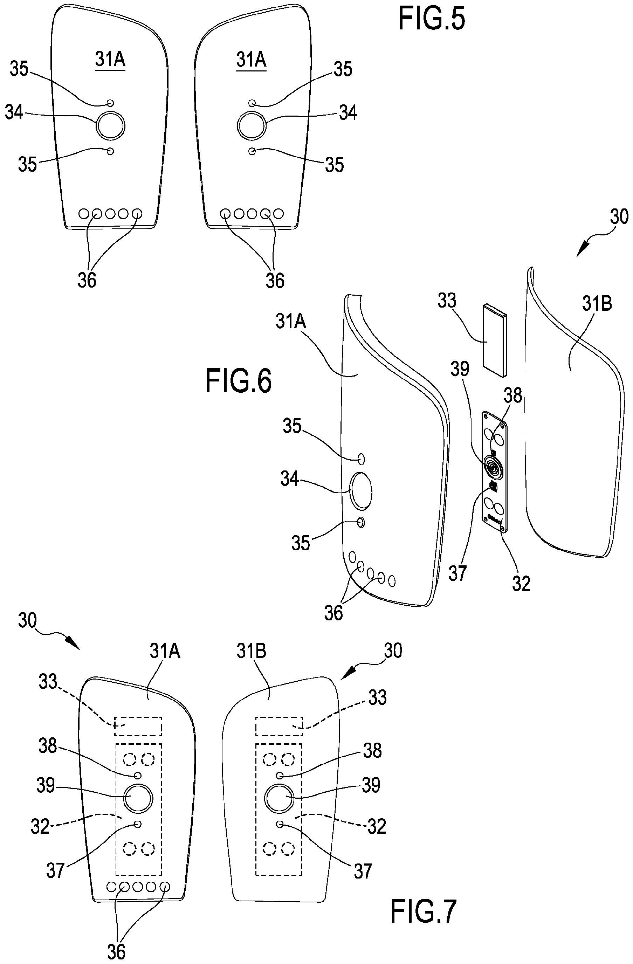

The device 10 is substantially formed by a sock 20, illustrated in figs 3,4, and by a shin guard 30, illustrated in figs 6,7.

The sock 20 illustrated in figs 3,4 is formed by a foot-shaped part 21 and a legging-shaped part 22.

The foot-shaped part 21 of the sock 20 is made of waterproof material. Inside the part 21 of the sock 20, four textile pressure sensors 23 are incorporated that are flexible, elastic and resistant, based on the variation of the electrical resistance of the material of which they are made if they are subjected to traction or compression. The four sensors 23 are arranged above at the instep. Each textile pressure sensor 23 has a substantially triangular shape. As can be seen easily in fig.4, the four sensors are arranged in a geometrically complementary manner. The sock 20 is also provided with an electric connection tongue 24 carrying a series of external electric contacts 25. The electric contacts 25 are connected to the sensors 23 through a series of electric conductors 26 incorporated into the foot-shaped part 21 of the sock 20.

The legging-shaped part 22 has at the front a pocket 27 closed on three sides and opened above. In the lower part the pocket 27 has a horizontal oblong opening 28.

The shin guard 30 illustrated in figs 6,7 is formed by a front half shell 31A and a rear half shell 31B, which are coupled together, between which an electronic board 32 and a battery 33 are inserted. The front half shell 31A, illustrated in fig.5, is of a material that is resistant to shocks and has centrally a circular opening 34 and two small circular holes 35, one above and the other below the opening 34, at the electronic board 32. The front half shell 31A, further, has a series of external contacts 36 below that are arranged horizontally. The external contacts 36 are connected electrically to the electronic board 32.

On the electronic board 32 the following components are mounted:
- a motion sensor with six or nine axes
- a sensor for detecting heart rate
- a sensor for detecting humidity
- a GPS antenna
- an antenna for transferring data to an external device
- a control unit
- a memory unit

On the electronic board 32 a pushbutton 37 for switching the electronic board 32 on or off and an indicator lamp 38 are also mounted. The pushbutton 37 is at the lower hole 35 whereas the indicator lamp 38 is at the upper hole 35

The electronic board 32 is connected for the necessary power to the battery 33, which can be a rechargeable lithium battery. For recharging, the electronic board is provided with a wireless charger 39 arranged at the opening 34.

With reference to figs 1,2, the shin guard 30 is inserted into the pocket 27 of the sock 20 and the contacts 25 of the tongue 24 of the sock 20 are joined to the contacts 36 of the shin guard 30 by magnetic coupling, so as to connect the sensors 23 electrically to the electronic board 32 and in particular to the control unit.

The monitoring device 10 formed by the combination of the sock 20 and of the shin guard 30 is intended to be worn by a soccer player to monitor his soccer activity.

The monitoring device 10 operates as follows.

Once the electronic board 32 is activated, the control unit of the electronic board 32 is able to monitor specific parameters relating to kicking the ball, as well as motion parameters and biomedical parameters.

For the specific soccer parameters, the pressure sensors 23 detect the impact of the foot of the soccer player with the ball, in particular going from the inside to the outside of the instep, the first sensor 23 detects the flat shot, the second sensor detects the inside-instep shot, the third and the fourth sensor 23 detect the outsideinstep shot. In this manner, for example, the number of times can be monitored that, during a match or a training session, the soccer player has kicked the ball, with which force and with which part of the foot.

With regard to the motion parameters, the GPS antenna and the motion sensor permit numerous detections of the motor performance of the soccer player. For example, it is possible to calculate the number of steps and kilometres travelled during the match or the training session, the average and maximum speed, the number of accelerations, the zones of the soccer pitch that were most covered, the zones of the soccer pitch in which the ball was hit more frequently, and more.

With regard to the biomedical parameters, the sensor for detecting the heart rate and the sensor for detecting the humidity enable the heart activity and the sweating of the soccer player to be monitored. For example, it is possible to detect the moments of greater cardiac stress to prevent heart problems and detect the sweating level to prevent cramps.

All the data gathered by the control unit of the electronic board 32 are sent by the antenna to an external device that, as shown in fig.2, can be a smartphone, indicated with S. Obviously, other external devices can be provided such as laptops, tablets, or other devices.

Data can be sent from the control unit of the electronic board 32 to the external device by a Bluetooth or Wi-Fi connection.

The external device can be used by a trainer to analyze the performance of the soccer player and, applied to all the players of a team, to analyze the performance of the team. And this can be used both in a match and during training.

It is clear that variations on and/or additions to what has been disclosed above and illustrated in the attached drawings can be provided.

The shape and the arrangement of the pressure sensors disclosed and illustrated is particularly effective. Nevertheless, variations in the shape, number and arrangement of the pressure sensors can be provided.

In particular, pressure sensors can be provided even at the toe of the sock to detect the toe shot of the soccer player, which is particularly used for example in 5-a-side soccer.

The pocket of the sock can be closed at the top by a flap fixed by an internal automatic button.

Instead of the pocket, other means can be provided to combine the shin guard with the sock, such as a system of hooking by guides, by snap coupling, by Velcro, or other system. The pocket is anyway a simple and effective system for combining the shin guard with the sock.

The shape and arrangement of the opening and of the holes of the shin guard can vary.

Instead of the electric connection tongue, other types of electrical connection can be provided to connect the pressure sensors to the control unit. For example the contacts connected to the pressure sensors can be made directly inside the pocket of the sock.

In one simplified embodiment, the biomedical parameter sensors can be omitted.

The monitoring device seen can be used to monitor performance in other sports in which a ball has to be hit with the foot, such as for example rugby and American football.

## Claims

1. Device (10) for monitoring sports performance, in particular soccer performance, comprising a sock (20), a shin guard (30) coupled with the sock (20), a series of pressure sensors (23), position sensors, motion sensors, a control unit connected to the pressure sensors (23), to the position sensors and to the motion sensors, and a data transmitter connected to an external device (S), wherein the control unit processes the data received from the pressure sensors (23), from the position sensors and from the motion sensors and transmits the data by the data transmitter to the external device, **characterized in that** the pressure sensors (23) are incorporated into the sock and the position sensors, the motion sensors, the control unit and the data transmitter are incorporated into the shin guard (30), wherein the sock (20) has a foot-shaped part (21) provided with the pressure sensors (23), wherein the pressure sensors (23) are arranged in a geometrically complementary manner in the foot-shaped part (21) of the sock (20) at the instep of the foot.

2. Monitoring device according to claim 1, wherein biomedical parameter sensors are also incorporated into the shin guard (30).

3. Monitoring device according to claim 1 or 2, wherein the pressure sensors are arranged in the foot-shaped part of the sock also at the toe of the foot.

4. Monitoring device according to any preceding claim, wherein the pressure sensors are textile pressure sensors (23) incorporated into the sock (20).

5. Monitoring device according to any preceding claim, wherein the pressure sensors (23) have a substantially triangular shape.

6. Monitoring device according to any preceding claim, wherein the sock (20) has a legging-shaped part (22) that has a front pocket (27) in which the shin guard (30) is received.

7. Monitoring device according to any preceding claim, wherein the sock (20) has a tongue (24) provided with contacts (25) and the shin guard (30) is provided with corresponding contacts (36), wherein the contacts (25) of the tongue (24) are connected to the pressure sensors (23) and the contacts (36) of the shin guard (30) are connected to the control unit, and wherein said contacts (25,36) are reciprocally connected in a disconnectable manner.

8. Monitoring device according to claim 6, wherein the sock (20) has a tongue (24) provided with contacts (25) and the shin guard (30) is provided with corresponding contacts (36), wherein the contacts (25) of the tongue (24) are connected to the pressure sensors (23) and the contacts (36) of the shin guard (30) are connected to the control unit, wherein said contacts (25,36) are reciprocally connected in a disconnectable manner, and wherein the pocket (27) has an opening (28) to permit said reciprocal connection between the contacts (25,36).

9. Monitoring device according to claim 6, wherein in the pocket contacts are obtained for direct connection between the pressure sensors and the control unit.

10. Monitoring device according to any preceding claim, wherein the shin guard (30) is provided with a pushbutton (37) accessible from the outside to switch the monitoring device on or off and is provided with an indicator lamp (38) visible from the outside to indicate the on or off status of the monitoring device.

11. Monitoring device according to any preceding claim, wherein a battery (33) for powering the sensors (23) and the control unit is incorporated into the shin guard (30).

12. Monitoring device according to claim 11, wherein a wireless battery charger (39) for recharging the battery (33) is incorporated into the shin guard (30).

13. Monitoring device according to any preceding claim, wherein data are transmitted to the outer device (S) by Bluetooth or Wi-Fi connection.

## Patentansprüche

1. Vorrichtung (10) zur Überwachung der sportlichen Leistung, insbesondere der fußballerischen Leistung, mit einem Stutzen (20), einem mit dem Stutzen (20) gekoppelten Schienbeinschoner (30), einer Reihe von Drucksensoren (23), Positionssensoren, Bewegungssensoren, einer mit den Drucksensoren (23), den Positionssensoren und den Bewegungssensoren verbundenen Steuereinheit und einem mit einer externen Vorrichtung (S) verbundenen Datensender, wobei die Steuereinheit die von den Drucksensoren (23), den Positionssensoren und den Bewegungssensoren empfangenen Daten verarbeitet und die Daten durch den Datensender an die externe Vorrichtung überträgt, **dadurch gekennzeichnet, dass** die Drucksensoren (23) in den Stutzen und die Positionssensoren, die Bewegungssensoren, die Steuereinheit und der Datensender in den Schienbeinschoner (30) eingebaut sind, wobei der Stutzen (20) einen fußförmigen Teil (21) aufweist, der mit den Drucksensoren (23) versehen ist, wobei die Drucksensoren (23) in geometrisch komplementärer Weise in dem fußförmigen Teil (21) des Stutzens (20) am Spann des Fußes angeordnet sind.

2. Überwachungsvorrichtung nach Anspruch 1, wobei Sensoren für biomedizinische Parameter auch in den Schienbeinschoner (30) eingebaut sind.

3. Überwachungsvorrichtung nach Anspruch 1 oder 2, wobei die Drucksensoren in dem fußförmigen Teil des Stutzens auch an der Fußspitze angeordnet sind.

4. Überwachungsvorrichtung nach einem vorstehenden Anspruch, wobei die Drucksensoren in den Stutzen (20) eingebaute Textildrucksensoren (23) sind.

5. Überwachungsvorrichtung nach einem vorstehenden Anspruch, wobei die Drucksensoren (23) eine im Wesentlichen dreieckige Form aufweisen.

6. Überwachungsvorrichtung nach einem vorstehenden Anspruch, wobei der Stutzen (20) einen leggingförmigen Teil (22) aufweist, der eine Vordertasche (27) hat, in der der Schienbeinschoner (30) aufgenommen ist.

7. Überwachungsvorrichtung nach einem vorstehenden Anspruch, wobei der Stutzen (20) eine mit Kontakten (25) versehene Lasche (24) aufweist und der Schienbeinschoner (30) mit entsprechenden Kontakten (36) versehen ist, wobei die Kontakte (25) der Lasche (24) mit den Drucksensoren (23) und die Kontakte (36) des Schienbeinschoners (30) mit der Steuereinheit verbunden sind, und wobei die Kontakte (25, 36) in lösbarer Weise miteinander verbunden sind.

8. Überwachungsvorrichtung nach Anspruch 6, wobei der Stutzen (20) eine mit Kontakten (25) versehene Lasche (24) aufweist und der Schienbeinschoner (30) mit entsprechenden Kontakten (36) versehen ist, wobei die Kontakte (25) der Lasche (24) mit den Drucksensoren (23) verbunden sind und die Kontakte (36) des Schienbeinschoners (30) mit der Steuereinheit verbunden sind, wobei die Kontakte (25, 36) auf lösbare Weise miteinander verbunden sind und wobei die Tasche (27) eine Öffnung (28) aufweist, um die gegenseitige Verbindung zwischen den Kontakten (25, 36) zu ermöglichen.

9. Überwachungsvorrichtung nach Anspruch 6, wobei in der Tasche Kontakte zur direkten Verbindung zwischen den Drucksensoren und der Steuereinheit erhalten werden.

10. Überwachungsvorrichtung nach einem vorstehenden Anspruch, wobei der Schienbeinschoner (30) mit einem von außen zugänglichen Druckknopf (37) versehen ist, um die Überwachungsvorrichtung ein- oder auszuschalten, und mit einer von außen sichtbaren Anzeigelampe (38) versehen ist, um den Ein- oder Ausschaltzustand der Überwachungsvorrichtung anzuzeigen.

11. Überwachungsvorrichtung nach einem vorstehenden Anspruch, wobei in den Schienbeinschoner (30) ein Akku (33) für die Stromversorgung der Sensoren (23) und der Steuereinheit eingebaut ist.

12. Überwachungsvorrichtung nach Anspruch 11, wobei in den Schienbeinschoner (30) ein drahtloses Ladegerät (39) zum Aufladen des Akkus (33) eingebaut ist.

13. Überwachungsvorrichtung nach einem vorstehenden Anspruch, wobei die Daten über eine Bluetooth- oder Wi-Fi-Verbindung an die externe Vorrichtung (S) übertragen werden.

## Revendications

1. Dispositif (10) pour surveiller des performances sportives, en particulier des performances de football, comprenant une chaussette (20), un protège-tibia (30) accouplé à la chaussette (20), une série de capteurs de pression (23), des capteurs de position, des capteurs de mouvement, une unité de commande connectée aux capteurs de pression (23), aux capteurs de position et aux capteurs de mouvement, et un émetteur de données connecté à un dispositif externe (S), dans lequel l'unité de commande traite les données reçues des capteurs de pression (23), des capteurs de position et des capteurs de mouvement et transmet les données par l'émetteur de données au dispositif externe, **caractérisé en ce que** les capteurs de pression (23) sont incorporés dans la chaussette et les capteurs de position, les capteurs de mouvement, l'unité de commande et l'émetteur de données sont incorporés dans le protège-tibia (30), dans lequel la chaussette (20) présente une partie en forme de pied (21) pourvue des capteurs de pression (23), dans lequel les capteurs de pression (23) sont agencés d'une manière complémentaire géométriquement dans la partie en forme de pied (21) de la chaussette (20) au niveau du cou-de-pied du pied.

2. Dispositif de surveillance selon la revendication 1, dans lequel des capteurs de paramètres biomédicaux sont également incorporés dans le protège-tibia (30).

3. Dispositif de surveillance selon la revendication 1 ou 2, dans lequel les capteurs de pression sont agencés dans la partie en forme de pied de la chaussette également au niveau de l'orteil du pied.

4. Dispositif de surveillance selon une quelconque revendication précédente, dans lequel les capteurs de pression sont des capteurs de pression textiles (23) incorporés dans la chaussette (20).

5. Dispositif de surveillance selon une quelconque revendication précédente, dans lequel les capteurs de pression (23) présentent une forme sensiblement triangulaire.

6. Dispositif de surveillance selon une quelconque revendication précédente, dans lequel la chaussette (20) présente une partie en forme de jambière (22) qui présente une poche avant (27) dans laquelle le protège-tibia (30) est reçu.

7. Dispositif de surveillance selon une quelconque revendication précédente, dans lequel la chaussette (20) présente une languette (24) pourvue de contacts (25) et le protège-tibia (30) est pourvu de contacts (36) correspondants, dans lequel les contacts (25) de la languette (24) sont connectés aux capteurs de pression (23) et les contacts (36) du protège-tibia (30) sont connectés à l'unité de commande, et dans lequel lesdits contacts (25, 36) sont connectés réciproquement de manière à pouvoir être déconnectés.

8. Dispositif de surveillance selon la revendication 6, dans lequel la chaussette (20) présente une languette (24) pourvue de contacts (25) et le protège-tibia (30) est pourvu de contacts (36) correspondants, dans lequel les contacts (25) de la languette (24) sont connectés aux capteurs de pression (23) et les contacts (36) du protège-tibia (30) sont connectés à l'unité de commande, dans lequel lesdits contacts (25, 36) sont connectés réciproquement de manière à pouvoir être déconnectés, et dans lequel la poche (27) présente une ouverture (28) pour permettre ladite connexion réciproque entre les contacts (25, 36).

9. Dispositif de surveillance selon la revendication 6, dans lequel dans la poche des contacts sont obtenus pour une connexion directe entre les capteurs de pression et l'unité de commande.

10. Dispositif de surveillance selon une quelconque revendication précédente, dans lequel le protège-tibia (30) est pourvu d'un bouton-poussoir (37) accessible depuis l'extérieur pour allumer ou éteindre le dispositif de surveillance et est pourvu d'un voyant lumineux (38) visible depuis l'extérieur pour indiquer l'état allumé ou éteint du dispositif de surveillance.

11. Dispositif de surveillance selon une quelconque revendication précédente, dans lequel une batterie (33) pour alimenter les capteurs (23) et l'unité de commande est incorporée dans le protège-tibia (30).

12. Dispositif de surveillance selon la revendication 11, dans lequel un chargeur de batterie sans fil (39) pour recharger la batterie (33) est incorporé dans le protège-tibia (30).

13. Dispositif de surveillance selon une quelconque revendication précédente, dans lequel des données sont transmises au dispositif extérieur (S) par une connexion Bluetooth ou Wi-Fi.
